(19) Europäisches Patentamt
European Patent Office
Office européen des brevets

(11) EP 2 324 811 B1

(12) **EUROPÄISCHE PATENTSCHRIFT**

(45) Veröffentlichungstag und Bekanntmachung des Hinweises auf die Patenterteilung:
**03.01.2018 Patentblatt 2018/01**

(51) Int Cl.:
***A61K 8/04*** *(2006.01)* ***A61K 8/44*** *(2006.01)*
***A61K 8/60*** *(2006.01)* ***A61K 8/86*** *(2006.01)*
***A61Q 5/10*** *(2006.01)* ***A61K 8/9706*** *(2017.01)*

(21) Anmeldenummer: **10187543.3**

(22) Anmeldetag: **14.10.2010**

(54) **Schaumförmige Färbemittel**

Foam-like dye

Colorant mousseux

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.11.2009 DE 102009046814**
**29.01.2010 DE 102010001360**

(43) Veröffentlichungstag der Anmeldung:
**25.05.2011 Patentblatt 2011/21**

(73) Patentinhaber: **Henkel AG & Co. KGaA**
**40589 Düsseldorf (DE)**

(72) Erfinder:
- **Kleen, Astrid**
  **20457, Hamburg (DE)**
- **Zirwen, Sabrina**
  **22089, Hamburg (DE)**
- **Akram, Mustafa**
  **22455, Hamburg (DE)**

(56) Entgegenhaltungen:
**EP-A1- 1 062 939**
**EP-A2- 1 284 135**
**EP-A2- 2 275 079**
**WO-A1-2009/054027**
**DE-A1-102005 056 158**
**GB-A- 2 361 247**

- **ANONYMOUS: "Synergistische Tensidmischungen", RESEARCH DISCLOSURE, MASON PUBLICATIONS, HAMPSHIRE, GB, Bd. 487, Nr. 1, 1. November 2004 (2004-11-01), XP007134443, ISSN: 0374-4353**
- **"International Cosmetic Ingredient Dictionary and Handbook", 2008, The Cosmetic, Toiletry, and Fragrance Association vol. 1, page 476,**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Mittel zum Färben, die eine spezielle Tensidkombination enthalten und geeignet sind durch spezielle Applikationsvorrichtungen in Form eines stabilen Schaums ausgetragen zu werden und ein Färbeverfahren unter Zuhilfenahme der Mittel.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Oxidative Färbemittel bestehen üblicherweise aus zwei Komponenten, deren Mischung ausreichend zähflüssig ist, damit sie bequem auf die Haare aufgetragen werden kann und dabei nicht tropft oder verläuft. Darüber hinaus gab es häufig Versuche, andere Konfektionierungsformen zu entwickeln. So wurde vorgeschlagen, dünnflüssigere Färbemittel mit speziellen Applikatorsystemen auf die Haare aufzubringen oder Färbemittel als Schaum zu applizieren. Bei der Schaumapplikation ist insbesondere der Einsatz von Aerosolschäumen verbreitet. Allerdings besteht in jüngerer Zeit auch das Bedürfnis, auf den Einsatz von Treibgasen verzichten zu können. In WO 2009/054027 A1 und US 2010/0251488 A1 werden Haarfärbeverfahren beansprucht, bei welchen ein durch Vermischen von zwei Komponenten hergestelltes Haarfärbemittel aus einem Nicht-Aerosol Schaumcontainer in Form eines Schaums auf den Haaren appliziert wird.

Ein weiteres Problem bei der Schaumapplikation ist die Stabilisierung der Schäume. Die Beschaffenheit von Schäumen ist dann als ideal zu bezeichnen, wenn bei der Produktabgabe ein fester, stabiler Schaum entsteht, der ein geschmeidiges Gefühl hinterlässt und auf dem Haar nur langsam bricht. Häufig ist aber zu beobachten, dass die ausgebrachten Schäume wenig stabil sind und schnell wieder in sich zusammenfallen, so dass eine dünnflüssige, tropfende Lösung zurückbleibt. Andererseits ist es auch wesentlich, dass der Schaum trotzdem die Haare gut benetzt, so dass ein guter Farbauftrag stattfindet kann. Die Schaumstabilität wird insbesondere durch die Anwesenheit größerer Mengen an Salzen und Farbstoff(vorprodukt)en negativ beeinflusst.

Aufgabe der vorliegenden Erfindung war es daher, Färbemittel für die Schaumapplikation, insbesondere ohne Verwendung von Treibgasen, zu optimieren, so dass die oben genannten Nachteile überwunden werden können.

Es wurde überraschenderweise gefunden, dass Färbemittel, die eine spezielle Tensidkombination sowie einen speziellen Gesamttensidgehalt enthalten, zu äußerst stabilen Schäumen führen, die eine einfache und intensive Färbung der Fasern ermöglichen. Die Stabilität bleibt auch bei höheren Salzkonzentrationen erhalten. Außerdem ermöglichen die erfindungsgemäßen Mittel intensivere Farbergebnisse und deutlich haltbare Farbergebnisse als bisher bekannte Schaumzubereitungen.

Ein erster Gegenstand der vorliegenden Erfindung sind daher Mittel zum Färben keratinischer Fasern, enthaltend in einem kosmetisch akzeptablen Träger mindestens ein Oxidationsfarbstoffvorprodukt sowie eine Kombination aus mindestens einem oder mehreren Zuckertensiden und mindestens einem oder mehreren amphoteren Tensiden, wobei das anwendungsbereite Mittel mindestens 10,5 Gew.-% Gesamttensidgehalt aufweist, das anwendungsbereite Mittel mindestens ein Oxidationsmittel enthält und das anwendungsbereite Mittel eine Viskosität von 0 bis 1000 mPas aufweist (gemessen bei 22°C im Brookfield-Viskosimeter Typ RV-T mit Spindel LV-1 beziehungsweise RV-1 und einer Geschwindigkeit von 30U/min).

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Als erste erfindungswesentliche Komponente enthalten die Mittel mindestens ein Oxidationsfarbstoffvorprodukt. Vorzugsweise enthält das Mittel eine oder mehrere Entwicklerkomponenten sowie gegebenenfalls eine oder mehrere Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Es kann erfindungsgemäß bevorzugt sein, als Entwicklerkomponente ein p-Phenylendiaminderivat oder eines seiner physiologisch verträglichen Salze einzusetzen. Besonders bevorzugte p-Phenylendiamine sind ausgewählt aus p-Phenylendiamin, p-Toluylendiamin, 2-Chlor-p-phenylendiamin, N,N-Bis-(2-hydroxyethyl)-p-phenylendiamin und N-Phenyl-p-phenylendiamin, 2-(2-Hydroxyethyl)-p-phenylendiamin, N-(4-Amino-3-methylphenyl)-N-[3-(1H-imidazol-1-yl)propyl]amin sowie ihren physiologisch verträglichen Salzen. Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten sind insbesondere: N,N'-Bis-(2-hydroxyethyl)-N,N'-bis-(4'-aminophenyl)-1,3-diamino-propan-2-ol und Bis-(2-hydroxy-5-aminophenyl)-methan und deren physiologisch verträglichen Salze. Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, und 4-Amino-3-methyl-phenol sowie deren

physiologisch verträglichen Salze.

Ferner kann die Entwicklerkomponente ausgewählt sein aus o-Aminophenol und seinen Derivaten, wie beispielsweise 2-Amino-5-methylphenol und dessen physiologisch verträglichen Salzen. Weiterhin kann die Entwicklerkomponente ausgewählt sein aus heterocyclischen Entwicklerkomponenten, wie beispielsweise den Pyridin-, Pyrimidin-, Pyrazol-, Pyrazolo-PyrimidinDerivaten und ihren physiologisch verträglichen Salzen.

Bevorzugte Pyrimidin-Derivate sind insbesondere 2,4,5,6-Tetraaminopyrimidin und 4-Hydroxy-2,5,6-triaminopyrimidin und deren physiologisch verträglichen Salze.

Ein bevorzugtes Pyrazol-Derivat ist 4,5-Diamino-1-(2-hydroxyethyl)-pyrazol und dessen physiologisch verträglichen Salze.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Enfinricklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Diese Gruppen stehen durch ein Doppelbindungssystem in Konjugation. Wenn die zyklische Verbindung ein Sechsring ist, so befinden sich die besagten Gruppen bevorzugt in ortho-Position oder meta-Position zueinander.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5- und 2,7-Dihydroxynaphthalin, 1-Acetoxy-2-methoxynaphthalin, Resorcin, 4-Chlor-resorcin und 2-Amino-3-hydroxypyridin und deren physiologisch verträglichen Salze.

Erfindungsgemäß bevorzugte Kupplerkomponenten sind

(A) m-Aminophenol und dessen Derivate wie beispielsweise 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivate, beispielsweise 2-Amino-5-ethylphenol,
(C) m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}amino)ethanol,
(D) o-Diaminobenzol und dessen Derivate,
(E) Di- beziehungsweise Trihydroxybenzolderivate wie beispielsweise 2-Methylresorcin und 1,2,4-Trihydroxybenzol,
(F) Pyridinderivate wie beispielsweise 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxypyridin,
(G) Naphthalinderivate wie beispielsweise 1-Naphthol und 2-Methyl-1-naphthol,
(H) Morpholinderivate wie beispielsweise 6-Hydroxybenzomorpholin,
(I) Chinoxalinderivate,
(J) Pyrazolderivate wie beispielsweise 1-Phenyl-3-methylpyrazol-5-on,
(K) Indolderivate wie beispielsweise 6-Hydroxyindol,
(L) Pyrimidinderivate oder
(M) Methylendioxybenzolderivate wie beispielsweise 1-(2'-Hydroxyethyl)-amino-3,4-methylendioxybenzol

sowie deren physiologisch verträglichen Salze.

Erfindungsgemäß besonders bevorzugte Kupplerkomponenten sind 1-Naphthol, 1,5- und 2,7-Dihydroxynaphthalin, 5-Amino-2-methylphenol, 2-Amino-3-hydroxypyridin, Resorcin, 4-Chlorresorcin, 2-Methylresorcin und 2,6-Dihydroxy-3,4-dimethylpyridin und deren physiologisch verträglichen Salze.

Die erfindungsgemäßen Mitteln enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen ihr Gesamtgewicht. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

Zur weiteren Nuancierung der resultierenden Farbtöne kann es erfindungsgemäß bevorzugt sein, dem Mittel weiterhin mindestens einen direktziehenden Farbstoff zuzusetzen. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna.

Heutzutage sind direktziehende Farbstoffe üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Als anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobezolsulfonsäuresäure-natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure-natriumsalz (C.I. 10,385; Acid Orange 3), 4-[(2,4-Dihydroxyphenyl)azo]-benzolsulfonsäure-natriumsalz (C.I. 14,270; Acid Orange 6), 4-[(2-Hydroxynaphth-1-yl)azo]-benzolsulfonsäure-natriumsalz (C.I. 15,510; Acid Orange 7), 4-[(2,4-Dihydroxy-3-[(2,4-dimethylphenyl)azo]-phenyl)azo]-benzolsulfonsäure-natriumsalz (C.I. 20,170; Acid Orange 24), 4-Hydroxy-3-[(2-methoxyphenyl)azo]-1-naphthalinsulfonsäure-natriumsalz (C.I. 14,710; Acid Red 4), 4-Hydroxy-3-[(4-sulfonaphth-1-yl)azo]-1-naphthalin-sulfonsäure-dinatriumsalz (C.I. 14,720; Acid Red No.14), 6-Hydroxy-5-[(4-sulfonaphth-1-yl)azo]-2,4-naphthalin-disulfonsäure-trinatriumsalz (C.I. 16,255; Ponceau 4R; Acid Red 18), 3-Hydroxy-4-[(4-sulfonaphth-1-yl)azo]-2,7-naphthalin-disulfonsäure-trinatriumsalz (C.I. 16,185; Acid Red 27), 8-Amino-1-hydroxy-2-(phenylazo)-3,6-naphthalin-disulfonsäure-dinatriumsalz (C.I. 17,200; Acid Red 33; Red 33), 5-(Acetylamino)-4-hydroxy-3-[(2-methylphenyl)azo]-2,7-naphthalin-disulfonsäure-dinatriumsalz (C.I. 18,065; Acid Red 35), 2-(3-Hydroxy-2,4,5,7-tetraiod-dibenzopyran-6-on-9-yl)-benzoesäure-dinatriumsalz (C.I. 45,430; Acid Red 51), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 8-[(4-(Phenylazo)phenyl)azo]-7-naphthol-1,3-disulfonsäure-dinatriumsalz (C.I. 27,290; Acid Red 73), 2',4',5',7'-Tetrabrom-3',6'- dihydroxyspiro[isobenzofuran-1(3H),9'-[9H]xanthen]-3-on-dinatriumsalz (C.I. 45,380; Acid Red 87), 2',4',5',7'-Tetrabrom-4,5,6,7-tetrachlor-3',6'- dihydroxyspiro[isobenzo-furan-1(3H),9'[9H]xanthen]-3-on-dinatriumsalz (C.I. 45,410; Acid Red 92), 3',6'-Dihydroxy-4',5'-diiodospiro[isobenzofuran-1(3H),9'(9H)- xanthen]-3-on-dinatriumsalz (C.I. 45425; Acid Red 95), 2-Hydroxy-3-((2-hydroxynaphth-1-yl)azo)-5-nitrobenzolsulfonsäure-natriumsalz (C.I. 15,685; Acid Red 184), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red 195), 3-Hydroxy-4-[(4-methyl-2-sulfonphenyl)-azo]-2-naphthalincarbonsäure-calciumsalz (C.I. 15,850:1; Pigment Red 57:1), 3-[(2,4-Dimethyl-5-sulfophenyl)azo]-4-hydroxy-1-naphthalin-sulfonsäure-dinatriumsalz (C.I. 14,700; Food Red No. 1; Ponceau SX; FD&C Red No. 4), 1,4- Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), Bis[4-(diethylamino)-phenyl](2,4-disulfophenyl)carbenium-inneres Salz, Natriumsalz (2:1) (C.I. 42,045; Food Blue No. 3; Acid Blue 1), Bis[4-(diethylamino)phenyl](5-hydroxy-2,4-disulfophenyl)-carbenium-inneres Salz, Calciumsalz (2:1) (C.I. 42,051; Acid Blue 3), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(phenylamino)-9,10-anthrachinon-2-sulfonsäure (C.I. 62,055; Acid Blue 25), 1-Amino-4-(cyclohexylamino)-9,10-anthrachinon-2-sulfonsäure-natriumsalz (C.I. 62045; Acid Blue 62), 2-(1,3-Dihydro-3-oxo-5-sulfo-2H-indol-2-yliden)-2,3-dihydro-3-oxo-1H-indol-5- sulfonsäure-dinatriumsalz (C.I. 73,015; Acid Blue 74), 9-(2-Carboxyphenyl)-3-[(2-methylphenyl)amino]-6-[(2-methyl-4-sulfophenyl)amino]xanthylium-inneres Salz, Natriumsalz (C.I. 45,190; Acid Violet 9), 1-Hydroxy-4-[(4-methyl-2- sulfophenyl)amino]-9,10-anthrachinon-natriumsalz (C.I. 60,730; D&C Violett No. 2; Acid Violet 43), Bis[3-nitro-4-[(4-phenylamino)-3-sulfo-phenylamino]-phenyl]-sulfon (C.I. 10,410; Acid Brown 13), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440; Food Black No. 1), 3',3",5',5"-Tetrabromphenolsulfonphthalein (Bromphenolblau).

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Als kationische direktziehende Farbstoffe eignen sich insbesondere 9-(Dimethylamino)-benzo[a]phenoxazin-7-ium-chlorid (C.I. 51,175; Basic Blue 6), Di[4-(diethylamino)phenyl][4-(ethylamino)naphthyl]carbenium-chlorid (C.I.

42,595; Basic Blue 7), Di-(4-(dimethylamino)phenyl)-(4-(methyl-phenylamino)-naphthalin-1-yl)carbenium-chlorid (C.I. 42,563; Basic Blue 8), 3,7-Di(dimethylamino)phenothiazin-5-ium-chlorid (C.I. 52,015 Basic Blue 9), Di[4-(dimethylamino)phenyl][4-(phenylamino)naphthyl] carbenium-chlorid (C.I. 44,045; Basic Blue 26), 2-[(4-(Ethyl(2-hydroxyethyl)amino)phenyl)azo]-6-methoxy-3-methyl-benzothiazolium-methylsulfat (C.I. 11,154; Basic Blue 41), 8-Amino-2-brom-5-hydroxy-4-imino-6-[(3-(trimethylammonio)phenyl)amino]-1(4H)-naphthalinon-chlorid (C.I. 56,059; Basic Blue No. 99), Bis[4- (dimethylamino)phenyl]-[4-(methylamino)phenyl]carbenium-chlorid (C.I. 42,535; Basic Violet 1), Tri(4-amino-3-methylphenyl)carbenium-chlorid (C.I. 42,520; Basic Violet 2), Tri[4-(dimethylamino)-phenyl]carbenium-chlorid (C.I. 42,555; Basic Violet 3), 2-[3,6-(Diethylamino)dibenzopyranium-9-yl]- benzoesäurechlorid (C.I. 45,170; Basic Violet 10), Di(4-aminophenyl)(4-amino-3- methylphenyl)carbeniumchlorid (C.I. 42,510 Basic Violet 14), 1,3-Bis[(2,4-diamino-5-methylphenyl)azo]-3-methylbenzol (C.I. 21,010; Basic Brown 4), 1-[(4-Aminophenyl)azo]-7-(trimethylammonio)- 2-naphthol-chlorid (C.I. 12,250; Basic Brown 16), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (C.I. 12,251; Basic Brown 17), 3-[(4-Amino-2,5-dimethoxyphenyl)azo]-N,N,N-trimethylbenzolaminiumchlorid (C.I. 12,605, Basic Orange 69), 3,7-Diamino-2,8-dimethyl-5-phenylphenazinium-chlorid (C.I. 50,240; Basic Red 2), 1,4-Dimethyl-5-[(4-(dimethylamino)phenyl)azo]-1,2,4-triazolium-chlorid (C.I. 11,055; Basic Red 22), 2-Hydroxy-1-[(2-methoxyphenyl)azo]-7-(trimethylammonio)-naphthalin-chlorid (C.I. 12,245; Basic Red 76), Di[4-(dimethylamino)phenyl]iminomethan-hydrochlorid (C.I. 41,000; Basic Yellow 2), 2-[2-((2,4-Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57), Bis[4-(diethylamino)phenyl]phenylcarbenium-hydrogensulfat (1:1) (C.I. 42,040; Basic Green 1), Di(4- (dimethylamino)phenyl)-phenylmethanol (C.I. 42,000; Basic Green 4), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid und direktziehende Farbstoffe, die einen Heterocyclus enthalten, der mindestens ein quaternäres Stickstoffatom aufweist.

Bevorzugte kationische direktziehende Farbstoffe sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die im Prioritätsdokument mit den Bezeichnungen (DZ1) bis (DZ9) offenbarten Verbindungen. Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Nichtionische direktziehende Farbstoffe:

Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere: 1,4-Bis[(2-hydroxyethyl)amino]-2-nitrobenzol, 1-(2-Hydroxyethyl)amino-2-nitro-4-[di(2-hydroxyethyl)amino]-benzol (HC Blue 2), 1-Methylamino-4- [methyl-(2,3-dihydroxypropyl)amino]-2-nitrobenzol (HC Blue 6),1-[(2,3- Dihydroxypropyl)-amino]-4-[ethyl-(2-hydroxyethyl)amino]-2-nitrobenzol-hydrochlorid (HC Blue 9), 1-[(2,3-Dihydroxypropyl)amino]-4-[methyl-(2-hydroxyethyl)amino]-2-nitrobenzol (HC Blue 10), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 2-((4-Amino-2-nitrophenyl)amino)-5-dimethylamino-benzoesäure (HC Blue 13), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2), 1-(2-Aminoethylamino)-4-[di(2-hydroxyethyl)amino]-2-nitrobenzol, 4-(Di(2-hydroxyethyl)amino)-2-nitro-1-phenylamino-benzol.

Geeignete rote Nitrofarbstoffe sind insbesondere: 1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)methylamino]-1-(methylamino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)-amino]-benzol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 4-(2,3-Dihydroxypropoxy)-1-[(2-hydroxyethyl)amino]-2-nitrobenzol (HC Orange 3), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3- Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, 7-Amino-3,4-dihydro-6-nitro-2H-1,4-benzoxazin (HC Red 14).

Geeignete gelbe Nitrofarbstoffe sind insbesondere:

1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[Di(2-hydroxyethyl)amino]-5-nitrophenol, 2-[(2-Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxy-propoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)-amino]-3-nitro-1-trifluormethyl-benzol (HC Yellow 13), 4-[(2-Hydroxyethyl)-amino]-3-nitro-benzonitril-(HC Yellow 14), 4-[(2-Hydroxyethyl)amino]-3-nitro-benzamid (HC Yellow 15) 3-[(2-Hydroxyethyl)-amino]-4-methyl-1-nitrobenzol, 4-Chlor-3-[(2-hydroxyethyl)amino]-1-nitrobenzol.

Geeignete Chinonfarbstoffe sind insbesondere:

1,4-Di[(2,3-dihydroxypropyl)amino]-9,10-anthrachinon, 1,4-Di[(2-hydroxyethyl)amino]-9,10-anthrachinon (C.I. 61,545, Disperse Blue 23), 1-[(2-Hydroxyethyl)amino]-4-methylamino-9,10-anthrachinon (C.I. 61,505, Disperse Blue 3), 2-[(2-Aminoethyl)amino]-9,10-anthrachinon (HC Orange 5), 1-Amino-4-hydroxy-9,10-anthrachinon (C.I. 60,710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (C.I. 75,470, Natural Red 4), 1-[(3-Aminopropyl)amino]-4-methylamino-9,10-anthrachinon (HC Blue 8), 1-[(3-Aminopropyl)-amino]-9,10- anthrachinon (HC Red 8), 1,4-Diamino-2-methoxy-9,10-anthrachinon (C.I. 62,015, Disperse Red 11, Solvent Violet No. 26), 1,4-Dihydroxy-5,8-bis[(2-hydroxyethyl)amino]-9,10-anthrachinon (C.I. 62,500, Disperse Blue 7, Solvent Blue No. 69), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-{6-[(3-Chlor-4-(methylamino)phenyl)imino]-4-methyl-3-oxo-1,4-cyclohexadien-1-yl}harnstoff (HC Red 9), 2-{{4-[Di(2-hydroxyethyl)amino]phenyl}amino}-5-[(2-hydroxyethyl)amino]-2,5-cyclohexadien-1,4-dion (HC Green 1), 5-Hydroxy-1,4-naphthochinon (C.I. 75,500, Natural Brown 7), 2-Hydroxy-1,4-naphthochinon (C.I. 75,480, Natural Orange 6), 1,2-Dihydro-2-(1,3-dihydro-3-oxo-2H-indol-2-yliden)-3H-indol-3-on (C.I. 73,000), 4-{{5-[(2-Hydroxyethyl) amino]-1-methyl-1H- pyrazol-4-yl}imino}-4,5-dihydro-5-[(2-hydroxyethyl)-imino]-1-methyl-1H-Pyrazol-sulfat(1:1), Hydrat(1:1).

Geeignete neutrale Azofarbstoffe sind insbesondere:

1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4- Aminophenyl)-azo]-1-[di(2-hydroxyethyl)amino]-3-methylbenzol (HC Yellow 7), 2,6-Diamino-3-[(pyridin-3-yl)azo]-pyridin, 2-{[4-(Acetylamino)phenyl]azo}-4-methylphenol (C.I. 11855; Disperse Yellow 3), 4-[(4-Nitrophenyl)azo]-anilin (C.I. 11,005; Disperse Orange 3).

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Weiterhin hat es sich als vorteilhaft erwiesen, wenn die erfindungsgemäßen Mittel zur weiteren Nuancierung zusätzlich eine oder mehrere Farbstoffvorstufen naturanaloger Farbstoffe enthalten. Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins der Formel (RN1),

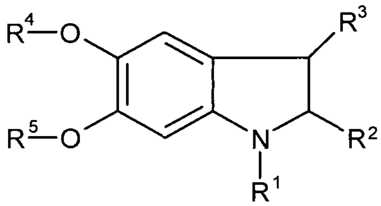

(RN1)

in der unabhängig voneinander

- $R^1$ steht für Wasserstoff, eine $C_1$-$C_4$-Alkylgruppe oder eine $C_1$-$C_4$-Hydroxy-alkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,

sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (RN2),

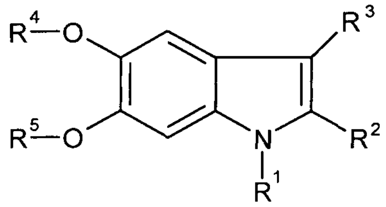

(RN2)

in der unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Als zweite erfindungswesentliche Komponente enthalten die erfindungsgemäßen Mittel ein oder mehrere Zuckertenside.

Gemäß einer ersten bevorzugten Ausführungsform enthält das Mittel als Zuckertensid mindestens ein Alkyl- oder Alkenyloligoglykosid. Diese Zuckertenside stellen bekannte nichtionische Tenside gemäß Formel dar,

$$R^1O\text{-}[G]_p$$

in der $R^1$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

Die Alkyl- und Alkenyloligoglykoside können sich von Aldosen beziehungsweise Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl ist. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest $R^1$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkyloligoglucoside auf Basis technischer $C_{9/11}$-Oxoalkohole (DP = 1 bis 3). Der Alkyl- bzw. Alkenylrest $R^1$ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

Gemäß einer zweiten Ausführungsform (C2) der Erfindung handelt es sich bei dem Zuckertensid um ein Fettsäure-N-alkylpolyhydroxyalkylamid, ein nichtionisches Tensid der Formel (III),

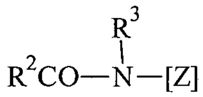

(III)

in der $R^2CO$ für einen aliphatischen Acylrest mit 6 bis 22 Kohlenstoffatomen, $R^3$ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Vorzugsweise leiten sich die Fettsäure-N-alkylpolyhydroxyalkylamide von reduzierenden Zuckern mit 5 oder 6 Kohlenstoffatomen, insbesondere von der Glucose, ab. Die bevorzugten Fettsäure-N-alkylpolyhydroxyalkylamide stellen daher Fettsäure-N-alkylglucamide dar, wie sie durch die Formel (IV) wiedergegeben werden:

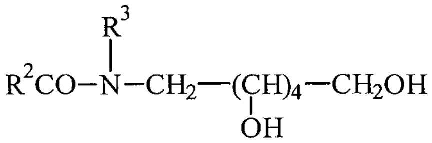

(IV)

Vorzugsweise werden als Fettsäure-N-alkylpolyhydroxyalkylamide Glucamide der Formel (IV) eingesetzt, in der $R^3$ für Wasserstoff oder eine Alkylgruppe steht und $R^2CO$ für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (IV), die durch reduktive Aminierung von Glucose mit Methylamin und anschließende Acylierung mit Laurinsäure oder $C_{12/14}$-Kokosfettsäure bzw. einem entsprechenden Derivat erhalten werden. Weiterhin können sich die Polyhydroxyalkylamide auch von Maltose und Palatinose ableiten.

Das beziehungsweise die Zuckertenside ist in den erfindungsgemäßen Mitteln bevorzugt in Mengen von 0,1 - 20 Gew.-%, bezogen auf das anwendungsbereite Mittel, enthalten. Mengen von 1 - 15 Gew.-% sind besonders bevorzugt. Mengen von 3 bis 8 Gew.-% sind ganz besonders bevorzugt.

Als dritte erfindungswesentliche Komponente enthalten die Mittel der vorliegenden Erfindung ein oder mehrere amphotere Tenside. Innerhalb der Gruppe der amphoteren Tenside sind sowohl die zwitterionischen als auch die ampholytischen Tenside erfindungsgemäß vorteilhaft.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$ - Gruppe tragen, wobei die Verbindungen, die eine $-COO^{(-)}$ tragen, erfindungsgemäß ganz besonders bevorzugt sind. Besonders geeignete zwitterionische Tenside sind die so genannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat:

Unter ampholytischen Tensiden werden solche oberflächenaktiven Verbindungen verstanden, die außer einer $C_8$ - $C_{24}$ - Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das $C_{12}$ - $C_{18}$ - Acylsarcosin.

In einer bevorzugten Ausführungsform der vorliegenden Erfindung wird ein amphoteres Tensid vom Betaintyp eingesetzt.

Besonders bevorzugt sind erfindungsgemäß zwitterionische Tenside der Formel (I),

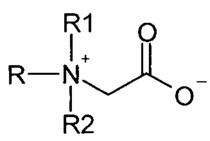

(I),

worin

| | |
|---|---|
| R | für eine gesättigte oder ungesättigte $C_{10}$-$C_{20}$-Alkylkette steht und |
| R1 und R2 | jeweils unabhängig voneinander für eine $C_1$-$C_4$-Alkylgruppe oder eine $C_2$-$C_4$-Hydroxyalkylgruppe stehen. |

Der Rest R steht dabei für eine Alkylkette mit 10 bis 20, vorzugsweise 10 bis 18 Kohlenstoffatomen, die gegebenenfalls eine oder mehrere Doppelbindungen ausweisen kann und gegebenenfalls verzweigt sein kann. Bevorzugte Beispiele solcher Alkylgruppen sind die Decyl-, Lauryl-, Myristyl-, Cetyl-, Palmoleyl-, 2-Hexyldecyl-, Stearyl-, Isostearyl-, Oleyl-, Elaidyl-, Petroselinyl-, Arachyl-, 2-Octyldodecyl oder Gadoleyl-Gruppe sowie deren Gemische, wie sie aufgrund von eingesetztem Rohstoff oder Herstellmethode anfallen. Bevorzugt sind für R Alkylgruppen auf Basis von Cocosalkyl- oder Talgfettalkylgruppen.

Die Reste R1 und R2 stehen unabhängig voneinander für eine $C_1$-$C_4$-Alkylgruppe oder eine $C_2$-$C_4$-Hydroxyalkylgruppe. Geeignete und bevorzugte $C_1$-$C_4$-Alkylgruppen sind dabei die Methyl-, Ethyl-, Propyl,- Isopropyl-, Butyl-, 1-Methylpropyl-, 2-Methylpropyl- und 1,1-Dimethylethyl-Gruppe. Geeignete und bevorzugte $C_2$-$C_4$-Hydroxyalkylgruppen sind dabei die 2-Hydroxyethyl-, 3-Hydroxypropyl- und 2-Hydroxypropyl-Gruppe. Besonders bevorzugt stehen R1 und R2 jeweils für eine Methylgruppe.

Ganz besonders bevorzugt ist es erfindungsgemäß, wenn das Mittel als zwitterionisches Tensid mindestens eine Verbindung der Formel (I) enthält, worin R1 und R2 jeweils für eine Methylgruppe und R für eine Cocosalkylgruppe stehen.

Solche besonders geeigneten zwitterionischen Tenside tragen die INCI-Bezeichnung Coco Betaine und werden beispielsweise als wässrige Lösung mit 30 Gew.-% Aktivsubstanz unter dem Handelsnamen Genagen® KB vertrieben.

Um einen stabilen Schaum zu erzeugen, ist es notwendig, dass das anwendungsbereite Mittel eine ausreichende Menge an zwitterionischem Tensid enthält. Eine Ausführungsform des vorliegenden Erfindung zeichnet sich dementsprechend dadurch aus, dass das Mittel ein oder mehrere zwitterionische Tenside der Formel (I) in einem Gesamtgewichtsanteil von mindestens 2,5 Gew.-%, bevorzugt von mindestens 3 Gew.-% und insbesondere bevorzugt von mindestens 4 Gew.-%, bezogen auf das Gesamtgewicht des anwendungsbereiten Mittels, enthält.

Die amphoteren Tenside sind in den Mittel vorzugsweise in Mengen von 0,1 bis 10 Gew.-%, insbesondere von 3 bis 8 Gew.-%, jeweils bezogen auf das anwendungsbereite Mittel enthalten. Weiterhin hat es sich als erfindungsgemäß als wesentlich zur Erzielung der gewünschten Schaumeigenschaften erwiesen, wenn das anwendungsbereite Mittel einen Gesamttensidgehalt von mindestens 10,5 Gew.-% aufweist. Mittel, die mindestens 11Gew.-%, vorzugsweise mindestens 12 Gew.-% Gesamttensidgehalt aufweisen sind insbesondere bevorzugt.

Weiterhin hat es sich als vorteilhaft erwiesen, wenn die erfindungsgemäßen Mittel mindestens ein weiteres nichtionisches Tensid enthalten, das von den Zuckertensiden verschieden ist.

Dabei hat es sich als besonders vorteilhaft erwiesen, wenn das weitere nichtionische Tensid einen HLB-Wert oberhalb von 10, vorzugsweise oberhalb von 14 aufweist. In dieser Gruppe wiederum sind die nichtionischen Tenside besonders bevorzugt, die Ethylenoxid- und/oder Propylenoxidgruppen enthalten. Ganz besonders bevorzugt sind nichtionische Tenside mit mehr als 29 Ethylenoxideinheiten. Neben den entsprechend ethoxylierten Fettalkoholen sind erfindungsgemäß insbesondere die Anlagerungsprodukte von 30 bis 60 Mol Ethylenoxid an Rizinusöl und gehärtetes Rizinusöl besonders geeignet. Diese speziellen nichtionischen Tenside ermöglichen eine weitere Verbesserung der Schaumkonsistenz, insbesondere im Hinblick auf eine höhere Festigkeit, eine gesteigerte Feinporigkeit und eine größere Geschmeidigkeit.

Außerdem hat es sich als vorteilhaft erwiesen, wenn die Anwendungszubereitung frei von kationischen Tensiden formuliert ist.

Ferner konnte in den dieser Erfindung zugrunde liegenden Arbeiten gezeigt werden, dass es vorteilhaft ist, wenn die Mittel neben den oben genannten Tensiden mindestens ein anionisches Tensid aufweisen. Als anionische Tenside sind

- lineare und verzweigte Fettsäuren mit 8 bis 30 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 8 bis 30 C-Atomen und x = 0 oder 1 bis 16 ist, und
- Alkylsulfate und Alkylpolyglykolethersulfate der Formel $R\text{-}O(CH_2\text{-}CH_2O)_x\text{-}OSO_3H$, in der R eine bevorzugt lineare Alkylgruppe mit 8 bis 30 C-Atomen und x = 0 oder 1 bis 12 ist,

erfindungsgemäß besonders bevorzugt.

In einer ganz besonders bevorzugten Ausführungsform der vorliegenden Erfindung enthalten die Mittel neben den erfindungswesentlichen Tensiden weiterhin ein nichtionisches Tensid, das von den Zuckertensiden verschieden ist sowie ein anionisches Tensid. Hinsichtlich der bevorzugten Vertreter der zusätzlichen Tenside in dieser Kombination sei an dieser Stelle auf die obigen Ausführungen verwiesen. Dabei hat es sich als vorteilhaft erwiesen, wenn das

anionische Tensid in der Oxidationsmittelzubereitung und die übrigen Tenside in der farbstoffhaltigen Komponente enthalten sind.

Überraschenderweise konnte gezeigt werden, dass die Anwesenheit geringer Mengen eines polymeren Verdickers die Schaumbeständigkeit positiv beeinflussen konnte. Daher ist es im Rahmen einer Ausführungsform der vorliegenden Erfindung bevorzugt, wenn die Mittel mindestens einen polymeren Verdicker enthalten.

Beispiele für erfindungsgemäß bevorzugte polymere Verdicker sind:

Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia

Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

Aus dieser umfangreichen Gruppe haben sich die Verdickungsmittel als besonders vorteilhaft erwiesen, die mindestens ein Monomer vom Typ der Acrylsäure oder Methacrylsäure sowie derer Derivate enthalten. Ein erfindungsgemäß ganz besonders bevorzugtes Polymer ist das unter der INCI-Bezeichnung Acrylates Copolymer bekannte Copolymer aus zwei oder mehr Monomeren, ausgewählt aus Acrylsäure, Methacrysäure und deren Ester mit $C_1$-$C_4$-Alkylgruppen.

Wie bereits ausgeführt, ist es besonders vorteilhaft, wenn der polymere Verdicker in geringen Mengen, vorzugsweise in Mengen von 0,05 bis 2 Gew.-%, insbesondere von 0,1 bis 1 Gew.-%, jeweils bezogen auf die anwendungsbereite Mischung, enthalten ist.

Eine weitere Verbesserung der pflegenden Eigenschaften des Produktes konnte durch den Einsatz eines Extraktes, der aus und/oder mithilfe von Algen und/oder Plankton gewonnen wird, erzielt werden. Insbesondere der Feuchtigkeitshaushalt der Fasern als auch deren Glanz konnte durch diese Extrakte erheblich gesteigert werden. Unter "Extrakten, die aus und/oder mithilfe von Algen und/oder Plankton gewonnen werden" sind erfindungsgemäß Wirkstoffmischungen zu verstehen, die entweder durch Extraktion von Algen und/oder Plankton selbst oder durch Extraktion der die Algen und/oder das Plankton umgebenen Wasserphase gewonnen werden.

Erfindungsgemäß bevorzugte Algen und/oder Planktonarten sind ausgewählt aus den Gattungen Haptophyta, Schlundgeißler (Cryptista), Euglenozoa, Dinozoa, Chlorarachniophyta, Goldalgen (Chrysophyta), Kieselalgen (Bacillariophyta, auch Diatomeen genannt), Braunalgen (Phaeophyta), Dinogellaten, Rotalgen (Rhodophyta), Grünalgen (Chlorophyta), Picobiliphyta sowie der Blaualgen (beispielsweise Oscillatoria und Spirulina)

Besonders bevorzugt sind im Rahmen der vorliegenden Erfindung Extrakte aus vornehmlich in Süßwasser vorkommenden Blaualgen.

Hinsichtlich der Art und Weise, wie die erfindungsgemäßen Extrakte aus den Algen- und/oder Planktonbestandteilen gewonnen wird, bestehen prinzipiell keine Einschränkungen.

Als Extraktionsmittel zur Herstellung der genannten Algenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Blaualgenextrakte, die mittels einer Wasser/Propylenglykol-Mischung gewonnen werden, haben sich als besonders geeignet erwiesen. Es hat sich dabei als besonders geeignet erwiesen, wenn diese Extraktionsmittel in einem Verhältnis von 1:10 bis 10:1 eingesetzt werden.

Weiterhin kann es erfindungsgemäß bevorzugt sein, Extrakte einzusetzen, die vor der Anwendung zumindest teilweise entfärbt wurden. Dies kann beispielsweise durch Nutzung von Aktivkohle erfolgen.

Ebenso ist möglich, in den erfindungsgemäßen Mitteln als Algen- oder Plankton-Extrakt die wässrige Aufzucht- beziehungsweise Kulturlösung von Algen oder Plankton einzusetzen. Dazu werden die Algen oder das Plankton zunächst mit einer physikalischen Trennmethode, wie Filtration oder Zentrifugation von der Kulturlösung abgetrennt.

In einer besonders bevorzugten Ausführungsform der vorliegenden Erfindung enthält das Mittel einen Extrakt einer Blaualge, vorzugsweise einer Süßwasserblaualge, ganz besonders bevorzugt einer Blaualge der Gattung Spirulina.

Die erfindungsgemäßen Mittel enthalten die Algen- und/oder Planktonextrakte vorzugsweise in einer Menge von 0,001 bis 5 Gew.-%, vorzugsweise von 0,01 bis 2 Gew.-% bezogen auf das anwendungsbereite Mittel.

In einer weiteren bevorzugten Ausführungsform enthalten die erfindungsgemäßen Mittel mindestens einen Wirkstoff, der aus Pflanzen der Gattung Aloe Vera gewonnen wird und für diese Pflanzengattung charakteristisch ist. Die Zugabe dieser Komponente ermöglicht eine überraschen intensive Verbesserung des Feuchtigkeitshaushaltes der Fasern.

Weiterhin kann es erfindungsgemäß bevorzugt sein, wenn die Mittel zusätzlich mindestens eine Aminosäure und/oder mindestens ein Protein. Erfindungsgemäß bevorzugte Aminosäuren sind Arginin, Serin, Lysin, Glycin, Tyrosin, Prolin, Glutamin, Cystein und Histidin. Der Aminosäuren und/oder die Proteine ermöglicht eine überraschend stark ausfallende Strukturgebung der Haare. Eine besonders gute Schaumqualität bei gleichzeitig guter Pflege der Haare kann überraschenderweise erzielt werden, wenn die Aminosäure Serin in den erfindungsgemäßen Mitteln eingesetzt wird.

Die Aminosäuren werden in den erfindungsgemäßen Mitteln vorzugsweise in einer Menge von 0,05 bis 5Gew.-%, insbesondere von 0,1 bis 2,5Gew.-% eingesetzt.

Im Rahmen einer weiteren bevorzugten Ausführungsform enthalten die erfindungsgemäßen Zubereitungen mindestens einen Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Moringa Olifeira, Grünem Tee, Eichenrinde, Brennnessel, Hamamelis, Hopfen, Henna, Kamille, Klettenwurzel, Schachtelhalm, Weißdorn, Lindenblüten, Mandel, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Moringa Olifeira, Grünem Tee, Eichenrinde, Brennnessel, Hamamelis, Hopfen, Kamille, Klettenwurzel, Schachtelhalm, Lindenblüten, Mandel, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Auch Extrakte aus Blüten haben sich erfindungsgemäß als besonders bevorzugt erwiesen. So können die Mittel beispielsweise einen Extrakt aus Seerosen, Orchideen, Wildrosen, Lotusblüten oder Kirschblüten enthalten. Dieser Extrakt kann vorzugsweise auch die Öl-haltige Fraktion dieser Pflanzen umfassen.

Ganz besonders geeignet sind die Extrakte aus Moringa Olifeira, Grünem Tee, Mandel, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Zubereitungen Mischungen aus mehreren, insbesondere aus zwei, verschiedenen Pflanzenextrakten einzusetzen.

In einer weiteren bevorzugten Ausführungsform enthält das erfindungsgemäße Mittel mindestens ein Pflanzenöl. Erfindungsgemäß bevorzugte Ölpflanzen sind Soja, Baumwolle, Raps, Erdnuss, Sonnenblume, Palmkern, Kokosnuss, Walnuss, Olive, Haselnuss, Weintraube, Aprikose, Pfirsich, Orange, Zitrone, Distel, Kürbiskern, Mais, Nachtkerze, Hanf, Leinsaat, Mohn, Sesam und Weizenkeim. Der Einsatz von pflanzlichen Ölen in den erfindungsgemäßen Mittel ermöglicht eine überraschende Steigerung des Glanzes der behandelten Haare. Insbesondere die Öle aus Aprikose, Pfirsich und Nachtkerze zeichnen sich diesbezüglich aus.

Weiterhin ist es erfindungsgemäß bevorzugt 2-Pyrrolidinon-5-carbonsäure und deren Derivate als einzusetzen. Besonders bevorzugt sind die Natrium-, Kalium-, Calcium-, Magnesium- oder Ammoniumsalze, bei denen das Ammoniumion neben Wasserstoff eine bis drei $C_1$- bis $C_4$-Alkylgruppen trägt. Das Natriumsalz ist ganz besonders bevorzugt. Die eingesetzten Mengen in den erfindungsgemäßen Mitteln betragen 0,05 bis 10 Gew.-%, bezogen auf die gesamte Anwendungszubereitung, besonders bevorzugt 0,1 bis 5Gew.-%, und insbesondere 0,1 bis 3 Gew.-%.

Ferner konnten die Erfinder der vorliegenden Anmeldung zeigen, dass es sich vorteilhaft auf die Beschaffenheit des Schaumes auswirkt, wenn die Mittel frei von Silikonölen formuliert sind.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Weiterhin konnten gute Kompromisse zwischen Schaumqualität und Pflegeeigenschaften der Färbemittel erzielt werden, wenn die Mittel weiterhin mindestens ein pflegendes Polymer viom Typ der amphoteren Polymere enthält. Bevorzugte amphotere Polymer werden in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannt.

Bevorzugt eingesetzte amphotere Polymere sind solche Polymerisate, die sich im wesentlichen zusammensetzen aus

(a) Monomeren mit quartären Ammoniumgruppen der allgemeinen Formel (II),

$$R^1\text{-}CH{=}CR^2\text{-}CO\text{-}Z\text{-}(C_nH_{2n})\text{-}N^{(+)}R^3R^4R^5\ A^{(-)} \qquad (II)$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoffatomen, Z eine NH-Gruppe oder ein Sauerstoffatom,

n eine ganze Zahl von 2 bis 5 und $A^{(-)}$ das Anion einer organischen oder anorganischen Säure ist, und
(b) monomeren Carbonsäuren der allgemeinen Formel (III),

$$R^6\text{-CH=CR}^7\text{-COOH} \qquad (III)$$

in denen $R^6$ und $R^7$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet. Ein derartiges Polymer wird beispielsweise unter der INCI-Bezeichnung Acrylamidopropyltrimonium Chloride/Acrylates Copolymer im Handel angeboten.

Die erfindungsgemäßen Mittel enthalten die amphoteren Polymere bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Die erfindungsgemäßen Mittel können die Inhaltsstoffe in einem geeigneten wässrigen, alkoholischen oder wässrig-alkoholischen Träger enthalten. Es ist aber auch möglich, eine der beiden Komponenten als pulverförmige oder auch Tabletten-förmige Formulierung bereitzustellen. Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen.

Die erfindungsgemäßen Mittel können erfindungsgemäß weitere organische Lösemittel, wie beispielsweise Monoethanolamin, Methoxybutanol, Benzylalkohol, Ethyldiglykol oder 1,2-Propylenglykol, enthalten. Bevorzugt sind dabei alle wasserlöslichen organischen Lösemittel. Monoethanolamin ist ein ganz besonders bevorzugtes organisches Lösemittel.

Bevorzugte erfindungsgemäße Mittel sind dadurch gekennzeichnet, dass sie zusätzlich ein nichtwässriges Lösungsmittel enthalten, wobei besonders bevorzugte erfindungsgemäße Mittel das Lösungsmittel in einer Konzentration von 0,1 - 30 Gewichtsprozent, bevorzugt in einer Konzentration von 1 - 20 Gewichtsprozent, ganz besonders bevorzugt in einer Konzentration von 2 - 10 Gewichtsprozent, jeweils bezogen auf das Mittel, enthalten.

In weiter bevorzugten erfindungsgemäßen Mitteln ist das Lösungsmittel ausgewählt aus Monoethanolamin, Ethanol, n-Propanol, Isopropanol, n-Butanol, Propylenglykol, n- Butylenglykol, Glycerin, Diethylenglykolmonoethylether, Diethylenglykolmono-n- butylether , Phenoxyethanol und Benzylalkohol sowie ihren Mischungen.

Die Formulierung in Wasser-basierten Lösungen, die frei von Fett- und Ölkörpern sind, hat sich erfindungsgemäß als besonders vorteilhaft erwiesen.

Der pH-Wert der erfindungsgemäßen Mittel kann durch geeignete Inhaltstoffe wie Acidifizierungsmittel oder Alkalisierungsmittel in einem weiten Bereich eingestellt werden.

In einer besonders bevorzugten Ausführungsform werden die erfindungsgemäßen Mittel dabei frei von Ammoniak als Alkalisierungsmittel formuliert. "Frei von Ammoniak" bedeutet dabei erfindungsgemäß, dass der Ammoniakgehalt der erfindungsgemäßen Mittel unterhalb von 1 Gew.-%, vorzugsweise unterhalb von 0,5 Gew.-%, insbesondere unterhalb von 0,1Gew.-%, jeweils bezogen auf das anwendungsbereite Mittel, liegt.

Zur Einstellung eines alkalischen pH-Wertes wird erfindungsgemäß vorzugsweise ein Alkanolamin eingesetzt. Erfindungsgemäß einsetzbare Alkanolamine werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methyl-propan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Erfindungsgemäß ganz besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Besonders bevorzugte Mittel enthalten als Alkanolamin mindestens Monoethanolamin.

Bevorzugt sind die Alkanolamine in einer Menge von 0,05 bis 20 Gew.-%, insbesondere von 0,5 bis 15 Gew.-%, jeweils bezogen auf das Gesamtgewicht des anwendungsbereiten Mittels, enthalten.

Bei einer Anwendung von Oxidationsmitteln wird das eigentliche Färbemittel unmittelbar vor der Anwendung durch Mischung der Zubereitung des Oxidationsmittels mit der Zubereitung, enthaltend die Verbindungen das mindestens eine Oxidationsfarbstoffvorprodukt, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu.

Weiterhin hat es sich als erfindungswesentlich erwiesen, dass die resultierende Applikationsflüssigkeit dünnflüssig formuliert ist. Erfindungsgemäß sind Applikationsflüssigkeiten, die nach Mischung mit der Oxidationsmittelzubereitung eine Viskosität von 0 bis 1000 mPas aufweisen (gemessen bei 22°C im Brookfield-Viskosimeter Typ RV-T mit Spindel LV-1 beziehungsweise RV-1 und einer Geschwindigkeit von 30U/min). Eine Viskosität von 5 bis 500 mPas, insbesondere von 10 bis 50 mPas (gemessen untern den oben genannten Bedingungen) ist erfindungsgemäß ganz besonders bevorzugt.

Es ist erfindungsgemäß bevorzugt, wenn die Mittel der vorliegenden Erfindung mittels einer speziellen Applikationsvorrichtung, wie beispielsweise einer mechanischen Schäumvorrichtung, aufgeschäumt werden. Unter mechanischen Schäumvorrichtungen sind solche Vorrichtungen zu verstehen, welche das Verschäumen einer Flüssigkeit mit oder ohne Verwendung eines Treibmittels ermöglichen. Erfindungsgemäß sind insbesondere derartige Schäumvorrichtungen bevorzugt, die eine Schaumbildung durch Luft aus der Umgebung und insbesondere ohne Verwendung von Treibgas ermöglichen.

Als geeignete mechanische Schäumvorrichtung kann beispielsweise eine Schüttelflasche, ein handelsüblicher Pumpschäumer oder ein so genannter Squeeze-Schäumer verwendet werden. Während in einer Schüttelflasche die Schaumbildung durch mechanische Agitation der Applikationsflüssigkeit innerhalb der Flasche stattfindet, weisen sowohl Pump- als auch Squeeze-Schäumer einen speziell ausgebildeten Schaumkopf auf. Ein derartiger Schaumkopf weist eine Schaumkammer auf, in die die Applikationszubereitung gemeinsam mit Umgebungsluft eingezogen und dabei aufgeschäumt wird. Während beim Pumpschäumer die Applikationsflüssigkeit über einen Pumpmechanismus eingezogen wird, erfolgt dies bei dem Squeezeschäumer durch manuellen Druck auf den Flaschenkörper, dessen elastische Wandungen sich reversibel zusammendrücken lassen. Der Squeezeflaschenkörper weist am oberen Ende eine Öffnung auf, die in eine Verschäumvorrichtung mündet. Der Squeezeflaschenkörper besitzt bevorzugt ein inneres Volumen von 100 bis 200 mL. Der Squeezeflaschenkörper wird bevorzugt derart mit dem oxidativen Färbemittel befüllt, so dass mindestens 20 % des Innenvolumens, besonders bevorzugt mindestens 30 % des Innenvolumens mit Luft gefüllt ist. Dabei hat es sich als vorteilhaft erwiesen, wenn die Oxidationsmittelzubereitung bereits ab Werk in dem Squeezeflaschenkörper konfektioniert ist und die Oxidationsfarbstoffzubereitung erst unmittelbar vor der Anwendung hinzugefügt wird. Dazu hat es sich als vorteilhaft erwiesen, wenn der Schaumkopf mittels eines Schraubmechanismus mit dem Squeezeflaschenkörper verbunden ist, so dass der Applikator an dieser Stelle zwecks Zugabe der Oxidationsfarbstoffzubereitung geöffnet werden kann.

Bevorzugt werden die Wandungen der erfindungsgemäßen Applikatorsysteme aus einem Polyolefin, wie beispielsweise Polypropylen (PP), high density Polyethylen (HDPE), medium density Polyethylen (MDPE), low density Polyethylen (LDPE), linear low density Polyethylen (LLDPE), gefertigt. Darunter ist Polypropylen (PP) bevorzugt geeignet.

Die Verschäumvorrichtung umfasst bevorzugt eine Mischkammer, mindestens eine Schaumerzeugungseinrichtung und einen Schaumkanal. Letzterer ist gegebenenfalls unterteilt in einen Schaumführungskanal und Schaumaustrittskanal. Die Öffnung des Squeezeflaschenkörper grenzt üblicherweise an die Mischkammer. Die Schaumerzeugungseinrichtung befindet sich üblicherweise zwischen Mischkammer und Schaumkanal.

Die Mischkammer weist üblicherweise einen Einlass für die flüssige Zusammensetzung aus dem Squeezeflaschenkörper und einen Einlass für Luft aus dem Squeezeflaschenkörper auf. Alternativ kann aber auch Luft aus der Umgebung des Applikators angesaugt werden. Die Zuführung des flüssigen oxidativen Färbemittels zum Einlass in die Mischkammer erfolgt bevorzugt durch ein Steigrohr, welches mit einem Ende am Einlass der Mischkammer befestigt ist und mit seinem anderen Ende in die flüssige Zusammensetzung eintaucht. Vorzugsweise ist das Steigrohr so lang, dass es bis zum Boden des Squeezeflaschenkörpers reicht, so dass eine vollständige Entnahme des Produktes möglich wird.

Die Schaumerzeugungseinrichtung ist bevorzugt derart in der Verschäumvorrichtung angeordnet, dass bei Durchführung eines Pumpvorganges am Pumpbehälter oder Squeezevorganges am Flaschenkörper Luft und das flüssige Oxidationsfärbemittel gleichzeitig in die Mischkammer gelangen und von dort aus durch die Schaumerzeugungseinrichtung hindurch in den Schaumkanal aufschäumen. Die Schaumerzeugungseinrichtung umfasst dabei bevorzugt mindestens ein poröses Material, wie z.B. eine poröse Keramik, oder besonders bevorzugt mindestens ein Netz. Liegt die Schaumerzeugungseinrichtung als Netz vor, so weist das Netz bevorzugt eine Lochdichte von 50 bis 220 mesh (mesh = Anzahl der Löcher pro Inch), besonders bevorzugt von 90 bis 200 mesh, ganz besonders bevorzugt von 125 bis 175 mesh auf.

Im Rahmen einer besonders bevorzugten Ausführungsform wird bei Durchführung eines Pump- oder Squeezevorganges Luft und das flüssige Oxidationsfärbemittel gleichzeitig in die Mischkammer befördert und von dort aus durch eine erste Schaumerzeugungseinrichtung in Form eines Netzes gedrückt, wobei der entstandene Schaum in den Schaumführungskanal gelangt, wo er vor Austritt aus dem Applikator durch eine zweite Schaumerzeugungseinrichtung in Form eines Netzes in den Schaumaustrittskanal gedrückt wird. Das erste Netz weist bevorzugt eine Lochdichte von 50 bis 220 mesh (mesh = Anzahl der Löcher pro Inch), besonders bevorzugt von 90 bis 200 mesh, ganz besonders

bevorzugt von 125 bis 175 mesh auf. Das zweite Netz weist bevorzugt eine Lochdichte von 160 bis 280 mesh, besonders bevorzugt von 175 bis 245 mesh und ganz besonders bevorzugt von 180 bis 220 mesh, auf.

Erfindungsgemäß geeignete Pumpschäumer werden beispielsweise von der Firma Airspray unter der Handelsbezeichnung "Airfoamer" angeboten. Insbesondere bevorzugt ist der Airfoamer mit der Typenbezeichnung G3.

Auch Squeezefoamer sind prinzipiell bereits aus dem Stand der Technik bekannt. Erfindungsgemäß geeignete Schäumer sind beispielsweise von der Firma Supermatic Kunststoff AG in ihrem Internetauftritt www.squeeze-foamer.com beschrieben.

Die Anwendungstemperaturen des resultierenden Schaums können in einem Bereich zwischen 15 und 40 °C liegen. Nach einer Einwirkungszeit von 5 bis 45 Minuten wird das Haarfärbemittel durch Ausspülen von dem zu färbenden Haar entfernt.

Erfindungsgemäß bevorzugt ist auch der Einsatz von so genannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mind. "zweizähnig" ist. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab.

Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze.

Im Rahmen der vorliegenden Erfindung können vorzugsweise Komplexbildner der folgenden chemischen Gruppen einzeln oder im Gemisch miteinander eingesetzt werden:

a) Polycarbonsäuren, bei denen die Summe der Carboxyl- und gegebenenfalls Hydroxylgruppen mindestens 5 beträgt wie Gluconsäure,

b) stickstoffhaltige Mono- oder Polycarbonsäuren wie Ethylendiamintetraessigsäure (EDTA), N-Hydroxyethylethylendiamintriessigsäure, Diethylentriaminpentaessigsäure, Hydroxyethyliminodiessigsäure, Nitridodiessigsäure-3-propionsäure, Isoserindiessigsäure, N,N-Di-(β-hydroxyethyl)glycin, N-(1,2-Dicarboxy-2-hydroxyethyl)-glycin, N-(1,2-Dicarboxy-2-hydroxyethyl)asparaginsäure oder Nitrilotriessigsäure (NTA),

c) geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppenhaltige Derivate hiervon,

Als Polycarbonsäuren a) werden im Rahmen dieser Patentanmeldung Carbonsäuren -auch Monocarbonsäuren- verstanden, bei denen die Summe aus Carboxyl- und den im Molekül enthaltenen Hydroxylgruppen mindestens 5 beträgt. Komplexbildner aus der Gruppe der stickstoffhaltigen Polycarbonsäuren, insbesondere EDTA, sind bevorzugt. Bei den erfindungsgemäß erforderlichen alkalischen pH-Werten der Behandlungslösungen liegen diese Komplexbildner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt.

Eine weitere Substanzklasse mit komplexbildenden Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und OctaNatriumsalz der DTPMP, eingesetzt. Als Komplexbildner wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Diese Stoffe werden nachstehend beschrieben.

Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Auch Dipicolinsäure wird erfindungsgemäß vorzugsweise als Komplexbildner eingesetzt. Mittel, die eine Kombination aus einem EDTA-Salz und, HEDP und Dipicolinsäure enthalten sind erfindungsgemäß ganz besonders bevorzugt.

Ein zweiter Gegenstand der vorliegenden Erfindung ist ein Verfahren zur Färbung keratinischer Fasern, bei dem ein erfindungsgemäßes Mittel aus einer Applikationsvorrichtung als Schaum ausgebracht wird, dieser auf den Fasern verteilt wird, dann für eine Zeit von 1 bis 60 Minuten, vorzugsweise von 5 bis 40 Minuten, auf den Fasern verbleibt und anschließend aus den Fasern ausgewaschen wird.

Dabei kann der ausgebrachte Schaum unmittelbar auf den Haaransatz aufgetragen und anschließend mit den Händen oder einem mechanischen Hilfsmittel auf den Fasern verteilt werden. Es ist aber auch denkbar, dass der Schaum zunächst auf ein mechanisches Hilfsmittel, wie beispielsweise einen Kamm, aufgebracht wird und anschließend mit dessen Hilfe auf den Fasern verteilt wird. Unabhängig von der Art der Auftragung kann es erfindungsgemäß bevorzugt sein, wenn der Schaum anschließend in die Haare einmassiert wird.

Es ist erfindungsgemäß bevorzugt, wenn die Applikationszubereitung bis unmittelbar vor der Anwendung als 2-Komponentensystem konfektioniert ist und erst bei der Anwendung durch Mischung der Komponenten hergestellt wird.

Dementsprechend ist es bevorzugt, wenn das erfindungsgemäße Mittel gemäß Anspruch 1 unmittelbar vor der Anwendung durch Mischung eines ersten Mittels (A), enthaltend das mindestens eine Oxidationsfarbstoffvorprodukt, und eines zweiten Mittels (B), enthaltend mindestens ein Oxidationsmittel, erhalten wird.

Bezüglich weiterer bevorzugter Ausführungsformen der erfindungsgemäßen Verfahren gilt mutatis mutandis das zu den erfindungsgemäßen Mitteln Gesagte.

Besonders bevorzugt ist es, die Ausfärbung durch physikalische Maßnahmen zu unterstützen. Erfindungsgemäße Verfahren, bei denen die Anwendung durch Einwirkung von Wärme und/oder UV-Strahlung während der Einwirkzeit unterstützt wird.

Beispiele

Es wurden die folgenden Zubereitungen hergestellt. Die Mengenangaben verstehen sich, soweit nichts anderes vermerkt ist, jeweils in Gewichtsprozent.

*1 Färbelösung*

| Rohstoff | Erfindungsgemäße Färbelösung | Nichterfindungsgemäße Färbelösung |
|---|---|---|
| Plantacare® 818 UP | 20,0 | 20,0 |
| Dehyton® K | 26,0 | 8,0 |
| Genamin® STAC | -- | 1,0 |
| Cremophor® CO 60 | 3,0 | 0,5 |
| Tetranatrium-EDTA | 0,2 | 0,2 |
| p-Toluylendiaminsulfat | 3,35 | 3,35 |
| 2-Amino-4-(2-hydroxyethyl)aminoanisol-sulfat | 0,15 | 0,15 |
| Resorcin | 1,2 | 1,2 |
| m-Aminophenol | 0,4 | 0,4 |
| Natriumsulfit | 0,2 | 0,2 |
| Ascorbinsäure | 0,05 | 0,05 |
| Monoethanolamin | 10,0 | 1,0 |
| Ammoniak (25%ig) | -- | 6,0 |
| Eau Vitale d'algue bleue® | 2,0 | 2,0 |
| Wasser | ad 100 | ad 100 |

*2 Entwicklerzubereitung*

| Rohstoff | Menge |
|---|---|
| Dipicolinsäure | 0,1 |
| Dinatriumpyrophosphat | 0,03 |
| Aculyn® 33A | 2,5 |
| Texapon® NSO | 2,0 |
| Wässrige Natronlauge (45%ig) | 0,5 |
| Turpinal® SL | 1,5 |
| Wasserstoffperoxid (wässrig, 50 %ig) | 15,0 |
| Wasser | ad 100 |

*3 Verzeichnis der eingesetzten Handelsprodukte*

| | |
|---|---|
| Aculyn® 33A | Acrylpolymer (ca. 28% Festkörper in Wasser; INCI-Bezeichnung: Acrylates Copolymer) |
| Cremophor® CO60 | hydriertes Rizinusöl mit ca. 60 EO-Einheiten (INCI-Bezeichnung: PEG-60 Hydrogenated Castor Oil) (BASF) |
| Dehyton® K | N,N-Dimethyl-N-(C8-18-kokosamidopropyl)ammoniumaceto-betain (ca. 30% Aktivsubstanz; INCI-Bezeichnung: Aqua (Water), Cocamidopropyl Betaine) (Cognis) |
| Eau Vitale d'algue bleue® | Blaualgenextrakt (ca. 0,1-0,99Gew.-% Aktivsubstanzgehalt; INCI-Bezeichnung: Aqua (Water), Plankton Extract, Penoxyethanol) (Soliance) |
| Plantacare® 818UP | C8-14-Alkylpolyglucosid (ca. 51-53% Akticsubstanzgehalt; INCI-Bezeichnung: Coco-Glucoside, Aqua (Water)) (Cognis) |
| Texapon® NSO | Laurylethersulfat, Natriumsalz (ca. 27,5% Aktivsubstanz; INCI-Bezeichnung: Sodium Laureth Sulfate) (Cognis) |
| Turpinal® SL | 1-Hydroxyethan-1,1-diphosphonsäure (ca. 58 - 61% Aktivsubstanzgehalt; INCI-Bezeichnung: Etidronic Acid, Aqua (Water)) (Solutia) |

*4 Ausfärbung*

4.1 Anwendung mit einer Squeeze-Schäumer

60ml der Oxidationsmittelzubereitung wurde in einer Squeeze-Flasche mit Schaumkopf vorgelegt. Unmittelbar vor der Anwendung wurden 60ml der erfindungsgemäßen Färbelösung vorsichtig zugegeben, so dass keine Schaumbildung erfolgte. Anschließend wurde die Squeezeflasche mehrfach vorsichtig auf den Kopf und wieder zurückgedreht, so dass eine gründliche Durchmischung der Komponente bei geringer Schaumbildung stattfand. Die resultierende Anwendungszubereitung hatte eine Viskosität von 15mPas (gemessen bei 22°C im Brookfield-Viskosimeter Typ RV-T mit Spindel LV-1 und einer Geschwindigkeit von 30U/min) und wies einen pH-Wert von 9,5 auf.

Der Schaum wurde durch Drücken der Squeeze-Flasche direkt auf den Haaransatz ausgebracht, anschließend die Haarlängen benetzt und zum Schluss mit einem Kamm gleichmäßig in den Fasern verteilt. Nach einer Einwirkzeit von 30 Minuten bei Raumtemperatur wurden die Haare gründlich mit Wasser gespült, shampooniert und mit einem Haartrockner getrocknet.

Die Haare waren satt schwarzbraun gefärbt, zeigten eine perfekte Farb-Egalisierung von Wurzel bis Haarspitze und einen hervorragenden Glanz.

4.2 Anwendung mit einer Pump-Schäumer

60ml der Oxidationsmittelzubereitung wurde in einer Pump-Flasche mit Schaumkopf vorgelegt. Unmittelbar vor der Anwendung wurden 60ml der Färbemittelzubereitung vorsichtig zugegeben, so dass keine Schaumbildung erfolgte. Anschließend wurde die Pump-Flasche mehrfach vorsichtig auf den Kopf und wieder zurückgedreht, so dass eine gründliche Durchmischung der Komponente bei geringer Schaumbildung stattfand. Die resultierende Anwendungszubereitung hatte eine Viskosität von 12mPas (gemessen bei 22°C im Brookfield-Viskosimeter Typ RV-T mit Spindel LV-1 und einer Geschwindigkeit von 30U/min) und wies einen pH-Wert von 9,5 auf.

Der Schaum wurde durch Benutzung des Pumpkopfes zunächst auf die Handinnenfläche und anschließend auf den Haaransatz und die Haarlängen ausgebracht, zum Schluss mit einem Kamm gleichmäßig in den Fasern verteilt. Nach einer Einwirkzeit von 30 Minuten bei Raumtemperatur wurden die Haare gründlich mit Wasser gespült, shampooniert und mit einem Haartrockner getrocknet.

Die Haare waren satt schwarzbraun gefärbt, zeigten eine perfekte Farb-Egalisierung von Wurzel bis Haarspitze und einen hervorragenden Glanz.

4.3 Vergleichsversuche

4.3.1 Durchführung

Jeweils 60ml der Oxidationsmittelzubereitung wurden in zwei Squeeze-Flasche mit Schaumkopf vorgelegt. Unmittelbar vor der Anwendung wurden zu den Proben 60ml der erfindungsgemäßen Färbelösung beziehungsweise 60ml der nicht-erfindungsgemäßen Färbelösung vorsichtig zugegeben, so dass keine Schaumbildung erfolgte. Anschließend wurden die Squeezeflaschen mehrfach vorsichtig auf den Kopf und wieder zurückgedreht, so dass eine gründliche Durchmischung der Komponente bei geringer Schaumbildung stattfand.

Der Schaum wurde durch Drücken der Squeeze-Flasche auf Haarsträhnen (Kerling Naturhaar 3-0) ausgebracht und mit einem Kamm gleichmäßig in den Fasern verteilt. Nach einer Einwirkzeit von 30 Minuten bei Raumtemperatur wurden die Strähnen gründlich mit Wasser gespült, shampooniert und mit einem Haartrockner getrocknet.

4.3.2 Messmethodik

Die Strähnen wurden nach dem Färbevorgang an jeweils 4 Messpunkten farbmetrisch vermessen und die Ergebnisse gemittelt. Die Ergebnisse der Messungen wurden mithilfe des CIELAB Farbenraums quantifiziert.

Der L-Wert steht dabei für die Helligkeit der Färbung (schwarz-weiß-Achse); je größer der Wert für L ist, desto heller ist die Färbung.

Der a-Wert steht für die rot-grün-Achse des Systems; je größer der Wert ist, umso mehr ist die Färbung ins Rote verschoben.

Der b-Wert steht für die gelb-blau-Achse des Systems; je größer der Wert ist, umso mehr ist die Färbung ins Gelbe verschoben.

Farbunterschiede werden mittels des ΔE-Wertes charakterisiert, der entsprechend Gleichung (I) berechnet wird:

$$\Delta E = \sqrt{(\Delta L)^2 + (\Delta a)^2 + (\Delta b)^2} \qquad (I)$$

4.3.3 Ergebnisse

Es ergaben sich die folgenden Messergebnisse:

| | L | a | b |
|---|---|---|---|
| Erfindungsgemäße Färbelösung | 15,4 | 0,7 | 0,0 |
| NichtErfindungsgemäße Färbelösung | 16,8 | 1,5 | 1,5 |

Bereits an dem niedrigeren L-Wert kann abgelesen werden, dass die Färbung mit den erfindungsgemäßen Mittel deutlich intensiver ausfällt als die Färbung mit der Vergleichslösung. Der Farbunterschied ΔE zwischen den beiden Färbungen beträgt 2,2 und ist somit bereits mit dem bloßen Auge deutlich wahrzunehmen.

## Patentansprüche

1. Mittel zum Färben keratinischer Fasern, enthaltend in einem kosmetisch akzeptablen Träger mindestens ein Oxidationsfarbstoffvorprodukt sowie eine Kombination aus mindestens einem oder mehreren Zuckertensiden und mindestens einem oder mehreren amphoteren Tensiden, **dadurch gekennzeichnet, dass** das anwendungsbereite Mittel mindestens 10,5 Gew.-% Gesamttensidgehalt aufweist,
dass das anwendungsbereite Mittel mindestens ein Oxidationsmittel enthält und
dass das anwendungsbereite Mittel eine Viskosität von 0 bis 1000 mPas aufweist (gemessen bei 22°C im Brookfield-Viskosimeter Typ RV-T mit Spindel LV-1 beziehungsweise RV-1 und einer Geschwindigkeit von 30U/min).

2. Mittel nach Anspruch 1, **dadurch gekennzeichnet, dass** es als Zuckertensid mindestens ein Alkylpolyglucosid enthält.

3. Mittel nach einem der Ansprüche 1 oder 2, **dadurch gekennzeichnet, dass** es als amphoteres Tensid mindestens ein Betainderivat enthält.

4. Mittel nach einem der Ansprüche 1 bis 3, **dadurch gekennzeichnet, dass** es mindestens ein weiteres nichtionisches Tensid enthält.

5. Mittel nach Anspruch 4, **dadurch gekennzeichnet, dass** das nichtionische Tensid einen HLB-Wert größer 10, vorzugsweise größer 14, aufweist.

6. Mittel nach Anspruch 4 oder 5, **dadurch gekennzeichnet, dass** das nichtionische Tensid mindestens 30 Ethylenoxideinheiten aufweist.

7. Mittel nach einem der Ansprüche 1 bis 6, **dadurch gekennzeichnet, dass** es weiterhin mindestens einen Extrakt enthält, der aus und/oder mithilfe von Algen und/oder Plankton gewonnen wird.

8. Mittel nach einem der Ansprüche 1 bis 7, **dadurch gekennzeichnet, dass** das Mittel frei von Ammoniak formuliert ist.

9. Verfahren zur Färbung keratinischer Fasern, **dadurch gekennzeichnet, dass** ein Mittel nach einem der Ansprüche 1 bis 8 aus einer Applikationsvorrichtung als Schaum ausgebracht wird, dieser auf den Fasern verteilt wird, dann für eine Zeit von 1 bis 60 Minuten, vorzugsweise von 5 bis 40 Minuten, auf den Fasern verbleibt und anschließend aus den Fasern ausgewaschen wird.

## Claims

1. An agent for dyeing keratin fibers, containing in a cosmetically acceptable carrier at least one oxidation dye precursor and a combination of at least one or more sugar surfactants and at least one or more amphoteric surfactants, **characterized in that**
the ready-to-apply agent has at least 10.5 wt.% total surfactant content,
the ready-to-apply agent contains at least one oxidizing agent, and
the ready-to-apply agent has a viscosity of from 0 to 1,000 mPas (measured at 22°C in an RVT Brookfield viscosimeter having the spindle LV-1 or RV-1 and a speed of 30 rpm).

2. The agent according to claim 1, **characterized in that** it contains at least one alkyl polyglucoside as the sugar surfactant.

3. The agent according to one of claims 1 or 2, **characterized in that** it contains at least one betaine derivative as the amphoteric surfactant.

4. The agent according to one of claims 1 to 3, **characterized in that** it contains at least one further non-ionic surfactant.

5. The agent according to claim 4, **characterized in that** the non-ionic surfactant comprises an HLB value of greater than 10, preferably greater than 14.

6. The agent according to claim 4 or 5, **characterized in that** the non-ionic surfactant comprises at least 30 ethylene

oxide units.

**7.** The agent according to one of claims 1 to 6, **characterized in that** it further contains at least one extract that is obtained from and/or by means of algae and/or plankton.

**8.** The agent according to one of claims 1 to 7, **characterized in that** the agent is formulated so as to be free of ammonia.

**9.** A method for dyeing keratin fibers, **characterized in that** an agent according to one of claims 1 to 8 is dispensed from an application device as a mousse, and said mousse is distributed over the fibers, then remains on the fibers for a period of from 1 to 60 minutes, preferably from 5 to 40 minutes, and is subsequently washed out of the fibers.

**Revendications**

**1.** Agent de coloration de fibres de kératine comprenant, dans un support cosmétiquement acceptable, au moins un précurseur de colorant d'oxydation et une combinaison d'au moins un ou de plusieurs tensioactifs de sucre et d'au moins un ou de plusieurs tensioactifs amphotères, **caractérisé en ce que**
l'agent prêt à l'emploi a une teneur totale en tensioactif d'au moins 10,5% en poids,
l'agent prêt à l'emploi contient au moins un agent oxydant, et
l'agent prêt à l'emploi a une viscosité de 0 à 1000 mPas (mesurée à 22°C dans un viscosimètre Brookfield de type RV-T avec une broche LV-1 ou RV-1 et à une vitesse de 30 T/min).

**2.** Agent selon la revendication 1, **caractérisé en ce qu'**il contient comme tensioactif de sucre au moins un polyglucoside d'alkyle.

**3.** Agent selon l'une des revendications 1 ou 2, **caractérisé en ce qu'**il contient comme tensioactif amphotère au moins un dérivé de la bétaïne.

**4.** Agent selon l'une des revendications 1 à 3, **caractérisé en ce qu'**il contient au moins un autre tensioactif non ionique.

**5.** Agent selon la revendication 4, **caractérisé en ce que** le tensioactif non ionique a une valeur HLB supérieure à 10, de préférence supérieure à 14.

**6.** Agent selon la revendication 4 ou 5, **caractérisé en ce que** le tensioactif non ionique comporte au moins 30 unités d'oxyde d'éthylène.

**7.** Agent selon l'une des revendications 1 à 6, **caractérisé en ce qu'**il contient en outre au moins un extrait obtenu à partir et/ou à l'aide d'algues et/ou de plancton.

**8.** Agent selon l'une des revendications 1 à 7, **caractérisé en ce que** l'agent est formulé sans ammoniaque.

**9.** Procédé de coloration de fibres de kératine, **caractérisé en ce qu'**un agent selon l'une des revendications 1 à 8 est délivre par un dispositif d'application sous forme de mousse, est réparti sur les fibres puis reste sur les fibres pendant une durée de 1 à 60 minutes, de préférence de 5 à 40 minutes, puis est éliminé des fibres par lavage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009054027 A1
- US 20100251488 A1
- EP 998908 A2
- GB 2104091 A
- EP 47714 A
- EP 217274 A
- EP 283817 A
- DE 2817369
- DE 3929973